# EUROPEAN PATENT APPLICATION

(11) **EP 4 679 096 A1**
(43) Date of publication of application: **14.01.2026**
(21) Application number: 24767035.9
(22) Date of filing: 01.03.2024
(51) Int. Cl.: G01N 33/68, C07K 14/47, G01N 27/62

(54) **BIOMARKERS AND METHOD FOR ESTIMATING BRAIN NEURODEGENERATIVE CONDITION**

(30) Priority: 03.03.2023 JP 2023032531
(71) Applicant: SHIMADZU CORPORATION, Kyoto-shi, Kyoto 604-8511 (JP); National Center for Geriatrics and Gerontology, Aichi 474-8511 (JP)
(72) Inventor: KANEKO, Naoki, Kyoto-shi, Kyoto 604-8511 (JP); NAKAMURA, Akinori, Obu-shi, Aichi 474-8511 (JP)
(74) Representative: Müller-Boré & Partner Patentanwälte PartG mbB
(86) International application number: PCT/JP2024/007691
(87) International publication number: WO 2024/185666

(57) **Abstract**

Provided are a biomarker capable of estimating a neurodegenerative condition by a simple method and an estimation method for the neurodegenerative condition. The biomarker for determining the brain neurodegenerative condition includes at least one selected from the group consisting of Ng53-75, Ng53-78, Ng48-75, Ng44-75 and Ng43-78 in the blood sample.

## Description

### TECHNICAL FIELD

The present invention relates to a biomarker and a method for estimating a brain neurodegenerative condition.

### BACKGROUND ART

The number of dementia patients is estimated to be about 50 million worldwide in 2017 and is expected to be about 82 million in 2030. In Japan, the number of dementia patients is estimated to reach 7 million in 2025. The causes of dementia are classified into neurodegenerative disorders of the brain, vascular dementia, and other etiologies. Among them, neurodegenerative disorders account for the largest proportion, and Alzheimer's disease (AD), which is widely recognized as one of the neurodegenerative disorders, accounts for more than half of all dementias. It has been reported that AB peptide can be used as a biomarker for determining the onset of Alzheimer's disease since amyloid β (AB) peptide generated by cleavage of amyloid precursor protein is deeply involved in the onset of Alzheimer's disease with respect to death of brain neurons (Non-Patent Documents 1 and 2 and Patent Documents 1 to 3).

Neurogranin (Ng) is a protein abundantly distributed in dendritic spines of brain neurons. Ng is a neuron-specific postsynaptic protein consisting of 78 amino acid residues, has a function of regulating calmodulin-dependent signaling, and is involved in synaptic plasticity. Non-Patent Document 3 and Non-Patent Document 4 report that Ng is also one of biomarkers of neurodegeneration and synaptic dysfunction, and that the concentration of Ng increases in cerebrospinal fluid (CSF) of AD patients. Non-Patent Document 5 reports that fragmentation of Ng is promoted in the brain of AD patients, and that post-translationally modified Ng such as acetylation and glutathionylation is present. In addition, it has also been reported that the ratio of the total value of the three types of post-translationally modified Ng1-78 to the Ng fragment peptide in CSF is increased in AD patients as compared with a standard sample. Non-Patent Documents 6 and 7 report that Ng48-76 in CSF is increased in AD patients as compared with a standard sample. Thus, Ng has been reported as a marker reflecting neurodegeneration of AD patients in CSF.

### PRIOR ART DOCUMENTS

### PATENT DOCUMENTS

Patent Document 1: JP 6424757 B2
Patent Document 2: JP 6410810 B2
Patent Document 3: JP 6467512 B2

### NON-PATENT DOCUMENTS

Non-Patent Document 1: Kaneko N, et al.: Novel plasma biomarker surrogating cerebral amyloid deposition. Proc Jpn Acad Ser B Phys Biol Sci. 2014; 90(9):353-364.

Non-Patent Document 2: Nakamura A, et al.: High performance plasma amyloid-B biomarkers for Alzheimer's disease. Nature. 2018; 554(7691):249-254.

Non-Patent Document 3: Portelius E, et al.: Cerebrospinal fluid neurogranin concentration in neurodegeneration: relation to clinical phenotypes and neuropathology. Acta Neuropathol. 2018; 136(3):363-376.

Non-Patent Document 4: Thorsell A, et al.: Neurogranin in cerebrospinal fluid as a marker of synaptic degeneration in Alzheimer's disease. Brain Res. 2010; 1362:13-22.

Non-Patent Document 5: Kvartsberg H, et al.: The intact postsynaptic protein neurogranin is reduced in brain tissue from patients with familial and sporadic Alzheimer's disease. Acta Neuropathol. 2019; 137(1):89-102.

Non-Patent Document 6: Kvartsberg H, et al.: Characterization of the postsynaptic protein neurogranin in paired cerebrospinal fluid and plasma samples from Alzheimer's disease patients and healthy controls. Alzheimers Res Ther. 2015 Jul 1; 7(1):40.

Non-Patent Document 7: Kvartsberg H, et al.: Cerebrospinal fluid levels of the synaptic protein neurogranin correlates with cognitive decline in prodromal Alzheimer's disease. Alzheimers Dement. 2015; 11(10):1180-90

### SUMMARY OF THE INVENTION

### PROBLEMS TO BE SOLVED BY THE INVENTION

A method for determining an AD patient using a biomarker in CSF involves extraction of CSF, and thus has high invasiveness to the patient. Accordingly, there is a demand for analysis by blood testing, which is minimally invasive and easily obtainable, as well as for a biomarker suitable for such testing. However, analysis of Ng and a fragment peptide thereof in blood has hardly progressed, and its efficacy as a biomarker for dementia having neurodegeneration has not been reported.

An object of the present invention is to provide a biomarker capable of estimating a neurodegenerative condition by a simple method and an estimation method for the neurodegenerative condition.

### MEANS FOR SOLVING THE PROBLEMS

The biomarker according to the first aspect of the present invention relates to a biomarker for determining a brain neurodegenerative condition, the biomarker including at least one selected from the group consisting of Ng53-75, Ng53-78, Ng48-75, Ng44-75 and Ng43-78 in a blood sample.

The estimation method for a brain neurodegenerative condition according to the first aspect of the present invention relates to an estimation method for a brain neurodegenerative condition, the method including: a measurement step of measuring a blood sample collected from a subject to obtain measured values of a plurality of neurogranin-related peptides; and a use step of using at least one measured value selected from the group consisting of Ng53-75, Ng53-78, Ng48-75, Ng44-75, and Ng43-78 among the measured values of the plurality of neurogranin-related peptides to determine a brain neurodegenerative condition.

### EFFECTS OF THE INVENTION

According to the biomarker and the estimation method of the first aspect of the present invention, the brain neurodegenerative condition of the patient can be easily estimated, and therefore, the biomarker and the estimation method can be used to determine whether dementia due to neurodegeneration has developed.

### BRIEF DESCRIPTION OF THE DRAWINGS

FIG. 1 shows a graph comparing normalized intensity (NI) of samples thawed, placed at room temperature for about 2 hours, and then re-frozen (samples under RT2h-FT1 conditions) with normalized intensity of samples not subjected to the process (samples under control conditions).
FIG. 2 shows a graph obtained by correcting the graph of FIG. 1 for samples under the RT2h-FT1 condition with the sum of NIs of a specific Ng peptide group (monovalent ions of 10 Ng peptides and divalent ions of 2 Ng peptides).
FIG. 3 shows a correlation plot diagram between an Ng peptide showing a high value in dementia and FDG-PET score. The vertical axis represents NI of each Ng peptide, and the horizontal axis represents FDG-PET score.
FIG. 4 shows a correlation plot diagram between an Ng peptide showing a low value in dementia and FDG-PET score.
FIG. 5 shows a correlation plot diagram between an Ng peptide showing a high value in dementia and VSRAD. The vertical axis represents NI of each Ng peptide, and the horizontal axis represents VSRAD.
FIG. 6 shows a correlation plot diagram between an Ng peptide showing a low value in dementia and VSRAD.
FIG. 7 shows a correlation plot diagram between an Ng peptide showing a high value in dementia and MMSE. The vertical axis represents NI of each Ng peptide, and the horizontal axis represents MMSE.
FIG. 8 shows a correlation plot diagram between an Ng peptide showing a low value in dementia and MMSE.

### MODE FOR CARRYING OUT THE INVENTION

### 1. First Embodiment

The estimation method according to the first embodiment is a method for determining a brain neurodegenerative condition, in which at least one selected from the group consisting of Ng53-75, Ng53-78, Ng48-75, Ng44-75, and Ng43-78 in a blood sample is used as a biomarker. This estimation method includes a measurement step and a use step in order.

### 1-1. Measurement Step

In the measurement step, a blood sample is measured to analyze the neurogranin-related peptide in the blood sample. As an example of a preferred measurement step of the first embodiment, purification treatment and mass spectrometry are sequentially performed on a blood sample. That is, after purification of a blood sample containing blood collected from a subject, mass spectrometry is performed to obtain the measured value of the neurogranin-related peptide in the blood sample. Hereinafter, this mode will be described in detail.

### (Purification Treatment)

The purification treatment of the blood sample preferably includes affinity purification. As a result, impurities included in the blood sample can be removed, and a trace amount of neurogranin-related peptide contained in the blood can be reliably detected.

In the first embodiment, the "neurogranin-related peptide" (hereinafter, abbreviated as "Ng peptide") includes, in addition to neurogranin (Ng1-78) (SEQ ID NO: 36) and a fragment peptide thereof, modified neurogranin such as acetylation or cysteinylation or a fragment peptide thereof. An example of an Ng peptide includes the peptides listed in Tables 3 and 4 in Examples described below.

The affinity purification may be performed once or multiple times, but the purification is preferably performed twice from the viewpoint of being able to detect the Ng peptide with higher sensitivity. In this case, the purification step includes a first bonding step, a first washing step, a first elution step, a neutralization step, a second bonding step, a second washing step, and a second elution step in this order.

The first bonding step involves bringing the blood sample into contact with the first carrier. As a result, the Ng peptide in the blood sample binds to the first carrier to obtain a first conjugate.

The blood sample is a sample solution containing blood collected from a subject, and preferably includes blood itself or a diluted solution thereof. The blood sample includes whole blood, plasma, and serum. The blood sample may be obtained by subjecting whole blood collected from an individual to treatment such as centrifugation and cryopreservation.

Examples of the diluted solution of the blood sample include a mixed solution of blood and a buffer solution. Examples of the buffer include a Tris buffer, a phosphate buffer, a HEPES buffer, and an ammonium acetate buffer.

For example, an organic solvent such as dimethylformamide or acetonitrile may be mixed with the diluted solution. The final concentration of the organic solvent is, for example, 5 to 30 (v/v)%. Thus, degradation of a desired Ng peptide over time can be suppressed while a blood sample collected from a subject is stored.

In addition, a surfactant may be mixed with the diluted solution. Examples of the surfactant include a neutral surfactant having a hydrophobic group having 7 or more and 15 or less carbon atoms. The concentration of the surfactant is, for example, 0.01 to 5 (v/v)%. As a result, non-specific adsorption to the first conjugate can be suppressed, and ionization interference in mass spectrometry can be reduced. Examples of such surfactants include surfactants having maltose in a hydrophilic moiety, such as n-nonyl-β-D-maltoside, n-nonyl-β-D-thiomaltoside, n-decyl-β-D-maltoside, and n-undecyl-β-D-maltoside (UDM); surfactants having trehalose in a hydrophilic moiety, such as, α-D-glucopyranosyl α-D-glucopyranoside monodecanoate (trehalose C10); and surfactants having glucose in a hydrophilic moiety, such as n-decyl-β-D-glucoside.

The blood sample is preferably further mixed with an internal standard substance in order to normalize or standardize the measured value. The internal standard substance preferably includes a peptide labeled with a stable isotope. The mixing amount of the internal standard substance is, for example, 1 pM or more and 1 nM or less with respect to the plasma sample.

The first carrier may be any carrier to which the Ng peptide can be bound, and examples thereof include an antibody-immobilizing carrier. The antibody immobilized on the first carrier is an antibody having an antigen binding site capable of recognizing the Ng peptide (anti-Ng peptide antibody), and examples thereof include an immunoglobulin having an antigen binding site capable of recognizing the Ng peptide or a fragment thereof. Examples of the immunoglobulin include IgG (IgG1, IgG2, IgG3, or IgG4), IgM, IgA, IgY, IgD, and IgE. Examples of the immunoglobulin fragment include F(ab')2, F(ab'), F(ab), Fd, Fv, L-chain, and H-chain. More specifically, clones NG2, NG7, EPR21152, fragments thereof, and the like are included.

The first washing step involves washing the first conjugate using the first washing solution after the first bonding step.

The first washing solution is preferably a neutral buffer containing the surfactant described above. This makes it possible to effectively remove highly hydrophobic undesirable components (for example, blood proteins, lipids, and glycolipids).

In the washing method, washing is preferably performed a plurality of times. For example, washing is performed using a neutral buffer containing a surfactant, followed by washing using a neutral buffer free of a surfactant. As the washing method, a general method may be employed, and examples thereof include a method of stirring a carrier in a washing solution and a method of spraying a washing solution from a washing nozzle. After washing with these neutral buffers, washing with water may be further performed, if necessary.

In the first elution step, the first conjugate is brought into contact with the first acidic solution after the first washing step. Accordingly, the Ng peptide is dissociated from the first conjugate, and the Ng peptide is eluted into the first acidic solution. As a result, a first eluate containing the Ng peptide is obtained.

Examples of the first acidic solution include an acidic aqueous solution (pH 0.5 to 3.5) such as a glycine buffer and hydrochloric acid. The first acidic solution preferably contains the surfactant described above. Accordingly, the Ng peptide can be more reliably dissociated from the first conjugate, and the detection sensitivity can be improved.

In the neutralization step, after the first elution step, the first eluate is mixed with a neutral buffer. As a result, the first eluate is neutralized to obtain a purified solution containing an Ng peptide.

The neutral buffer used in the neutralization step preferably contains the surfactant described above. Thus, non-specific adsorption to the second conjugate can be suppressed in the second bonding step.

The pH of the resulting purified solution is neutral, for example, pH 6.0 or more, preferably 6.5 or more, and is, for example, 8.5 or less, preferably 8.0 or less. Accordingly, in the second bonding step, the bonding efficiency can be improved.

The second bonding step involves bringing the purified solution into contact with the second carrier after the neutralization step. As a result, the Ng peptide in the purified solution binds to the second carrier to obtain a second conjugate.

The second carrier is preferably an antibody-immobilizing carrier, and specific examples thereof include the same antibody-immobilizing carriers as illustrated for the first carrier.

The second washing step involves washing the second conjugate using the second washing solution after the second bonding step.

The second washing solution is preferably a neutral buffer containing the surfactant described above. This makes it possible to effectively remove highly hydrophobic undesirable components.

As the washing method, a known method may be employed, and specifically, a method similar to the washing method illustrated in the first washing step may be performed.

In the second elution step, the second conjugate is brought into contact with the second acidic solution after the second washing step. Accordingly, the Ng peptide is dissociated from the second conjugate, and the Ng peptide is eluted into the second acidic solution. As a result, a second eluate containing the Ng peptide is obtained.

Examples of the acidic aqueous solution constituting the second acidic solution include those similar to the first acidic solution illustrated in the first elution step, and preferably include hydrochloric acid.

The second acidic solution preferably contains a volatile organic solvent. Accordingly, the Ng peptide can be efficiently dissociated from the second conjugate and eluted in the second acidic solution, and the recovery rate of the Ng peptide can be improved. Examples of the volatile organic solvent include organic solvents miscible with water in an arbitrary ratio, such as acetonitrile, methanol, ethanol, acetone, toluene, isopropanol, hexane, butanol, cyclohexane, ethylene glycol, benzene, chloroform, acetaldehyde, triethylamine, phenol, naphthalene, formaldehyde, tetrahydrofuran, and ethyl acetate.

The second acidic solution preferably further contains an amino acid such as methionine. As a result, the oxidation of the Ng peptide can be reduced, and the detection sensitivity can be improved before the Ng peptide is placed in the mass spectrometer and analysis is started.

The second acidic solution preferably further contains a protein of 9 kDa or more. Since the protein is contained in the mass spectrometry sample, the protein thus acts as a proton receptor during mass spectrometry, and monovalent ions of the Ng peptide can be efficiently generated. As a result, the detection sensitivity of the Ng peptide can be remarkably improved. The upper limit of the mass of the protein is, for example, 100 kDa or less, preferably 15 kDa or less. Specific examples of such proteins include bovine serum albumin (BSA), cytochrome, ovalbumin, and lysozyme.

### (Mass Spectrometry)

After the purification treatment, mass spectrometry is performed on the second eluate to detect the Ng peptide.

Examples of the ionization method in mass spectrometry include matrix assisted laser desorption/ionization (MALDI) and electrospray ionization (ESI), and atmospheric pressure chemical ionization (APCI). MALDI is preferably included from the viewpoint of enabling the detection of a trace amount of Ng peptide in a plasma sample with high sensitivity.

Examples of the mass spectrometry include, but are not limited to, time-of-flight mass spectrometry (TOF-MS), ion trap mass spectrometry (IT-MS), ion trap-time-of-flight mass spectrometry (IT-TOF-MS), and Fourier transform ion cyclotron mass spectrometry (FTICR-MS). When MALDI is used as the ionization method, the mass spectrometry is collectively referred to as MALDI-MS.

As a specific detection operation by MALDI-MS, for example, first, a matrix-containing solution is added to a MALDI plate dropwise and dried to arrange the matrix. Subsequently, the second eluate is added dropwise and dried on the matrix to obtain a MALDI-MS sample. Next, the MALDI-MS sample is irradiated with a laser to ionize the Ng peptide, and the ionized peptide is detected using a detector with the above apparatus and output as a mass spectrum.

Examples of the matrix include α-cyano-4-hydroxycinnamic acid, 2,5-dihydroxybenzoic acid, sinapinic acid, and 3-aminoquinoline. Preferably, a matrix additive is used in combination with the matrix. Examples of the matrix additive include phosphonic acid group-containing compounds, such as methylene diphosphonic acid, and ammonium salts. Examples of the solvent containing the matrix include acetonitrile, trifluoroacetic acid, methanol, ethanol, and water.

As a result, a measured value showing the peak intensity (including the peak area) of the ion peak corresponding to each of the plurality of Ng peptides is obtained together with the mass spectrum in which the plurality of Ng peptides contained in the blood sample is detected. Examples of such an Ng peptide include those in examples described later (see Tables 3 and 4). This measured value may be a relative intensity with the intensity of the reference peak taken as 100% or may be an intensity normalized (NI) by an internal standard. When a plurality of measured values (including normalized intensity) is obtained by dividing and measuring the second eluate into a multiple portions (a plurality of wells), the average value thereof may be used.

In addition, the Ng peptide detected by mass spectrometry may be detected as a peak of a monovalent ion, or may be detected at a peak of a monovalent ion and a polyvalent ion of divalent or more. For example, both ions are detected for Ng43-75. In this case, any biomarker may be used as the measured value of the biomarker, but in the case of MALDI-MS, preferably, the peak intensity detected at the peak of the monovalent ion is used for a small Ng peptide having a mass of less than 5000, and the peak intensity detected at the peak of the divalent ion is used for a large Ng peptide having a mass of 5000 or more. On the other hand, in the case of electrospray ionization (ESI), the peak intensity detected at the peak of the multivalent ion is preferably used.

### 1-2. Use Step

In the use step, at least one measured value selected from the group consisting of Ng53-75, Ng53-78, Ng48-75, Ng44-75, and Ng43-78 among the measured values of the plurality of Ng peptides obtained in the measurement step is used to determine the neurodegenerative condition. That is, a measured value of the Ng peptide serving as a biomarker is selected from many measured values of the Ng peptide detected in the mass spectrum obtained in the measurement step, and the measured value is used to determine the neurodegenerative condition as an index, a score, or the like.

In the first embodiment, the expression "measured value is used to determine the neurodegenerative condition" means that a measured value (including normalized and averaged values) in the Ng peptide serving as a biomarker (the measured value) may be used in part to determine the neurodegenerative condition. For example, (1) in addition to a case where the measured value is used as an index for determination as it is, (2) a case where the measured value is corrected or processed into a variable, and the correction value or the variable is used as an index for determination, (3) a case where the measured value of the Ng peptide other than the biomarker is added to the measured value, and the sum is used as an index for determination, (4) a case where a desired value or formula is added to, subtracted from, multiplied by or divided by the measured value, and the value is used as an index for determination, and (5) a combination thereof are included.

The Ng peptide serving as a biomarker is at least one selected from the group consisting of Ng53-75, Ng53-78, Ng48-75, Ng44-75, and Ng43-78. These sequences are shown in Table 1 below. The biomarkers may be used alone or in combination. The biomarker preferably includes Ng53-75, Ng53-78, Ng48-75, Ng44-75, Ng43-78, or a combination of Ng53-75 and Ng53-78. From the viewpoint of having high analysis accuracy (AUC) without correction (described later), a combination of Ng53-75, Ng53-78, Ng43-78, or Ng53-75 and Ng53-78 is more preferable. From the viewpoint of having high analysis accuracy for both Alzheimer's disease and non-Alzheimer's disease, a combination of Ng53-75, Ng43-78, or Ng53-75 and Ng53-78 is still more preferable.

**[Table 1]**

| Peptide name | Sequence | No. |
|---|---|---|
| Ng53-75 | RKGPGPGG PGGAGVARGG AGGGP | 1 |
| Ng53-78 | RKGPGPGG PGGAGVARGG AGGGPSGD | 2 |
| Ng48-75 | SGE RGRKGPGPGG PGGAGVARGG AGGGP | 3 |
| Ng44-75 | KKIKSGE RGRKGPGPGG PGGAGVARGG AGGGP | 4 |
| Ng43-78 | RKKIKSGE RGRKGPGPGG PGGAGVARGG AGGGPSGD | 5 |

The measured values may be used as correction values by performing correction processing, if necessary. In a correction method, for example, a value (defined as correction coefficient) may be calculated by summing the measured values of at least 2 Ng peptides obtained in the measurement step, and the measured value of each Ng peptide serving as a biomarker (including normalized intensity) may be then divided by the correction coefficient. In particular, the Ng peptide to be corrected for the measured value may be any of the Ng peptides illustrated above, among which Ng48-75 and Ng44-75 are preferably particularly corrected.

The Ng peptides subject to the correction coefficient may be at least two of Ng peptides detected in the measurement step. Preferably, at least two Ng peptides having an m/z (or mass) equal to or less than that of Ng38-75 are detected. Specifically, such Ng peptides preferably include at least two of Ng53-75, Ng52-75, Ng51-75, Ng53-78, Ng50-75, Ng48-75, Ng45-75, Ng44-75, Ng43-75, NNg43-77, Ng43-78, and Ng38-75. When a blood sample collected from a subject is stored at normal temperature or freeze-thawed, the biomarker is decomposed and reduced, and therefore, the measured value may vary due to the storage state of the blood sample. With this correction, the variation can be reduced, and the analysis can be performed more accurately.

When the measured value of the combination of Ng53-75 and Ng53-78 is used as the biomarker, correction may be performed, for example, after the measured value of Ng53-75 and the measured value of Ng53-78 are added up. Alternatively, after a correction value obtained by correcting the measured value of Ng53-75 alone and a correction value obtained by correcting the measured value of Ng53-78 alone are calculated, these correction values may be added up.

Subsequently, the neurodegenerative condition is determined using the measured values (including correction values) of the biomarkers.

Specifically, the measured values of the biomarkers in the blood sample collected from a subject are compared with a predetermined value. Then, when the measured value of the biomarker is higher or lower than the predetermined value, it is determined that neurodegeneration has occurred in the brain of the subject and dementia has developed in the subject. Examples of the mode of comparison include (1) a mode of comparing a measured value in a blood sample collected from a subject with a preset reference value that is a normal cognitive function, (2) a mode of collecting measured values of a plurality of blood samples collected over time (for example, every few months) from a subject and comparing these measured values of a plurality of times, and (3) a combination thereof. The reference value of (1) can be appropriately set from measurement values of blood samples collected from a plurality of persons with normal cognitive function and/or dementia in advance.

Among the biomarkers, Ng53-75, Ng53-78, Ng43-78, or the combination (sum) of Ng53-75 and Ng53-78 is increased in the blood of a subject in whom neurodegeneration has occurred. Therefore, in the aspect (1), when the measured value of the Ng peptide in the subject is higher than the reference value for normalizing the cognitive function, it is determined that neurodegeneration has occurred in the brain of the subject. In the aspect (2), when the measured value of the Ng peptide measured on a specific day is higher than the measured value of the Ng peptide measured on a day before the specific day, it is determined that neurodegeneration has occurred or progressed in the brain of the subject. On the other hand, in the opposite case, that is, in the aspects (1) and (2), when the former measured value is low, it is determined that neurodegeneration of the brain of the subject is suppressed.

On the other hand, Ng48-75 or Ng44-75 decreases in the blood of a subject in whom neurodegeneration has occurred. Therefore, in the aspect (1), when the measured value of the Ng peptide in the subject is lower than the reference value for normalizing the cognitive function, it is determined that neurodegeneration has occurred in the brain of the subject. In the aspect (2), when the measured value of the Ng peptide measured on a specific day is lower than the measured value of the Ng peptide measured before that day, it is determined that neurodegeneration has occurred or progressed in the brain of the subject. On the other hand, in the opposite case, that is, in the aspects (1) and (2), when the former measured value is high, it is determined that neurodegeneration of the brain of the subject is suppressed.

As described above, the estimation method using the biomarker of the first embodiment is a method for detecting and using an Ng peptide from a blood sample, in which a blood sample collected from a subject is measured to select and use the measured value of the specific Ng peptide from the measurement results, making it easy to estimate whether or not the brain neurodegenerative condition, that is, the functional loss of the brain neurons has occurred. For example, it is possible to easily predict the occurrence or progress of a loss of synaptic function, synaptic loss, and the like. In this method, the measured value can be thus used as information for diagnosing whether or not the subject has developed dementia caused by neurodegeneration. Specifically, the information can be used to diagnose dementia caused by neurodegeneration, including non-Alzheimer's dementia (dementia with Lewy bodies, frontotemporal dementia, etc.) in addition to representative Alzheimer's disease. Furthermore, the information can also be used as information for predicting the onset of dementia or diagnosing the onset risk of dementia.

This estimation method can also assess the effectiveness of the medical intervention. For example, if it is determined that the neurodegeneration has occurred in the subject, the implementation of the medical intervention is started, and then the estimation method is performed again to determine the condition of the neurodegeneration. Then, in performing the estimation method again, if it is determined that the progress of neurodegeneration is stopped, suppressed, or there is no neurodegeneration, it can be evaluated that the medical intervention is effective, and if it is determined that the neurodegeneration is progressing, it can be evaluated that the medical intervention is not effective. Furthermore, the present invention can also be applied to a case where it is determined that neurodegeneration has occurred, and thereafter, whether or not the neurodegeneration has progressed is followed up even in a state where there is no medical intervention.

### 1-3. Modified Example

(1) In the above embodiment, Ng53-75 alone, Ng53-78 alone, Ng48-75 alone, Ng44-75 alone, Ng43-78 alone, and a combination of Ng53-75 and Ng53-78 are adopted as the measured value, and each measured value is used for the determination of the neurodegenerative condition. However, for example, a variable may be calculated by multivariate analysis by combining the plurality of measured values, and this variable may be used for the determination of the neurodegenerative condition. That is, measured values including one or two selected from the group consisting of Ng53-75, Ng53-78, Ng48-75, Ng44-75, and Ng43-78 are selected to calculate these selected measured values as variables by multivariate analysis, and the variables may be used for the determination of the neurodegenerative condition. As a result, an area under curve (AUC) in ROC analysis is further improved, enabling more accurate decision-making.

As the multivariate analysis, a known mathematical method can be used, and examples thereof include discriminant analysis (linear discrimination method, secondary discrimination method, normalization discrimination method, etc.), multiple regression analysis, kernel method, principal component analysis, partial least squares regression, logistic regression, and Z-score.

(2) In the above embodiment, mass spectrometry is performed as the measurement step, that is, the measurement method is not limited as long as an Ng peptide group containing at least one selected from the group consisting of Ng53-75, Ng53-78, Ng48-75, Ng44-75, and Ng43-78 is used as a biomarker. For example, a sandwich immunoassay may be performed as a measurement method. In this method, an N-terminal-specific antibody of each of Ng53-75, Ng53-78, Ng48-75, Ng44-75, and Ng43-78 and an antibody recognizing a C-terminal region are sandwiched, whereby an Ng peptide to be the biomarker can be measured. In addition, the Ng peptide containing Ng53 as an N-terminal is mainly composed of Ng53-75 and Ng53-78, and the others are so minute that they cannot be detected by mass spectrometry and that they hardly affect the combined value of Ng53-75 and Ng53-78. Therefore, the combined value of Ng53-75 and Ng53-78 can be measured by sandwiching them between the N-terminal-specific antibody of Ng53-75 and the antibody that recognizes the C-terminal-side region from Ng53.

At this time, the measured value of each Ng peptide is appropriately determined according to the measurement method, but may be, for example, concentration, absorbance, or the like.

### 2. Aspect

It is understood by those skilled in the art that a plurality of exemplary embodiments described above are specific examples of the following aspects.

(Item 1) A biomarker for determining a brain neurodegenerative condition according to one aspect may be a biomarker for determining a brain neurodegenerative condition including at least one selected from the group consisting of Ng53-75, Ng53-78, Ng48-75, Ng44-75, and Ng43-78 in a blood sample.

(Item 2) In the biomarker according to Item 1, the biomarker may be selected from the group consisting of Ng53-75, Ng53-78, Ng48-75, Ng44-75, Ng43-78, and a combination of Ng53-75 and Ng53-78.

(Item 3) The estimation method for a brain neurodegenerative condition according to one aspect may include:
a measurement step of measuring a blood sample collected from a subject to obtain measured values of a plurality of neurogranin-related peptides; and
a use step of using at least one measured value selected from the group consisting of Ng53-75, Ng53-78, Ng48-75, Ng44-75, and Ng43-78 among the measured values of the plurality of neurogranin-related peptides for determining a brain neurodegenerative condition.

(Item 4) In the estimation method according to Item 3, in the use step, at least one measured value selected from the group consisting of Ng53-75,
Ng53-78, Ng48-75, Ng44-75, Ng43-78, and the sum of Ng53-75 and Ng53-78 may be used.

(Item 5) In the estimation method according to Item 3 or 4, in the use step, a total measured value of Ng53-75, Ng53-78, Ng43-78, or Ng53-75 and Ng53-78 may be used, and when the measured value is higher than a predetermined value, it may be determined that neurodegeneration has occurred in the brain of the subject.

(Item 6) In the estimation method according to Item 3 or 4, Ng48-75 or Ng44-75 may be used in the use step, and when the measured value is lower than a predetermined value, it may be determined that neurodegeneration has occurred in the brain of the subject.

(Item 7) In the estimation method according to any one of Items 3 to 6, the use step may be a step of comparing a preset reference value for the normal cognitive function with a measured value of the subject obtained by the measurement step.

(Item 8) In the estimation method according to any one of Items 3 to 6, the measurement step may be performed a plurality of times over time, and the use step may be a step of comparing a plurality of measured values obtained by performing the measurement step a plurality of times.

(Item 9) In the estimation method according to any one of Item 3 to 8, the measured value used in the use step may be a value corrected using a value obtained by adding at least two of the measured values of the plurality of neurogranin-related peptides measured in the measurement step.

(Item 10) In the estimation method according to any one of Item 3 to 9, in the use step, at least two measured values are selected from the group consisting of Ng53-75, Ng53-78, Ng48-75, Ng44-75, and Ng43-78 from among the measured values of the plurality of neurogranin-related peptides, a variable is calculated using multivariate analysis, and the variable may be used to determine a neurodegenerative condition.

(Item 11) In the estimation method according to any one of item 3 to 10, in the measurement step, mass spectrometry may be performed on the blood sample to obtain measured values of peak intensities for the plurality of neurogranin-related peptides.

(Item 12) In the estimation method according to any one of items 3 to 11, in the measurement step, affinity purification may be performed on the blood sample before mass spectrometry.

(Item 13) A method for detecting a neurogranin-related peptide, the method including the step of:
detecting at least one neurogranin-related peptide selected from the group consisting of Ng53-75, Ng53-78, Ng48-75, Ng44-75 and Ng43-78 in a blood sample.

### EXAMPLES

Next, the present invention will be described in detail with reference to Examples, but the scope of the present invention is not limited thereto.

### 1. Plasma Sample

At the National Center for Geriatrics and Gerontology, 161 plasma samples and clinical diagnostic information such as amyloid PET findings, FDG-PET findings, MRI findings and MMSE findings (Mini Mental State Examination) were acquired. Detailed information is shown in Table 2.

**[Table 2]**

| Clinical Diagnosis | | N | MMSE | FDG_PET | VSRAD | Amyloid PET (PIB tracer) | | | |
|---|---|---|---|---|---|---|---|---|---|
| | | | Average value | Average value | Average value | SUVR Average value | Aβ negative (N) | A*β* positive (N) | Other (N) |
| CN (Normal cognitive function) | | 62 | 28.9 | 0.53 | 0.75 | 1.24 | 49 | 13 | - |
| MCI (Mild cognitive impairment) | | 13 | 27.5 | 0.71 | 1.28 | 1.59 | 6 | 7 | - |
| Dementia | AD | 40 | 20.2 | 1.71 | 1.50 | 1.89 | 1 | 39 | - |
| | nonAD | 46 | 22.6 | 1.44 | 1.49 | 1.10 | 41 | 3 | Indeterminable 1 Not conducted 1 |

The subject is a case diagnosed with normal cognitive function (CN), mild cognitive impairment (MCI), Alzheimer's disease (AD), and dementia other than Alzheimer's disease (nonAD). A group of AD and nonAD was defined as a group of dementia.

FDG-PET findings were assessed using FDG-PET scores obtained by quantifying ¹⁸F-FDG-PET scans (see Herholz K, et al. ; Evaluation of a calibrated (18)F-FDG PET score as a biomarker for progression in Alzheimer disease and mild cognitive impairment. J Nucl Med. 2011 Aug; 52(8): 1218-26.).

As MRI findings, an index for evaluation of atrophy in the vicinity of the hippocampus by voxel-based morphometry using VSRAD was used (see Sone D, et al.; Japanese - Alzheimer's Disease Neuroimaging Initiative.Voxel-based Specific Regional Analysis System for Alzheimer's Disease (VSRAD) on 3-tesla Normal Database: Diagnostic Accuracy in Two Independent Cohorts with Early Alzheimer's Disease. Aging Dis. 2018 Aug 1; 9 (4): 755-760.).

PiB-PET images of the brains of the subjects were acquired in order to determine whether AB accumulation in the brain was positive or negative. A subject in which the accumulation amount of PiB in the cerebral cortex was larger than or equal to the accumulation amount of PiB in the non-specific white matter by visual interpretation evaluation by a nuclear medicine specialist was determined to be positive. A subject who had only non-specific accumulation in the white matter and hardly accumulated in the cortex was determined to be negative. The PiB accumulation average value (SUVR: Standard Uptake Value Ratio) quantified the PiB accumulation in the cortex, and the accumulation ratio of the cerebrum was determined on the basis of the cerebellum. AD cases confirmed to be AB-positive in amyloid PET were defined as AD_Aβ+ (N = 39), and nonAD cases confirmed to be AB-negative in amyloid PET were defined as nonAD_Aβ- (N = 41).

### <Experimental Example 1>

### [Preparation of Blood Sample]

DMSO (dimethyl sulfoxide) was mixed with a first buffer solution (0.1% n-undecyl-8-D-maltoside (UDM), 800 mM GlcNAc, 100 mM Tris-HCl, 300 mM NaCl; pH 7.4) containing a surfactant to prepare DMSO at a DMSO concentration of 20 (v/v)%. Subsequently, Ng45-75 (SIL-Ng45-75) and Ng24-75 (SIL-Ng24-75) labeled with a stable isotope as an internal standard were mixed with the first buffer so as to be 50 pM, thereby preparing an internal standard containing buffer. Subsequently, 200 µL of human plasma was mixed with 200 µL of an internal standard containing buffer, and the mixture was allowed to stand on ice for 5 to 60 minutes. Thus, a plasma sample was prepared. In SIL-Ng45-75 and SIL-Ng24-75, the carbon atoms and nitrogen atoms of some amino acids of Pro, Val, and Ala are substituted with ¹³C and ¹⁵N, respectively.

### [Purification]

### (First Bonding Step, First Washing Step, and First Elution Step)

Clone NG2 (manufactured by BioLegend, Inc.) of an anti-Ng antibody (IgG1) having 52-63 residues of human neurogranin (Ng) as an epitope was prepared. To 100 µg of an anti-Ng antibody, 5.5 mg of magnetic beads (Dynabeads M-270 Epoxy) were added in an immobilization buffer (0.1 M phosphate buffer containing 1.5 M ammonium sulfate; pH 7.4) at 37°C for 16 to 24 hours. Thus, antibody beads as an antibody-immobilizing carrier were produced.

Plasma samples were mixed with the antibody beads and incubated at 4°C for 1 hour. The antibody beads were then washed once with 100 µL of the first wash buffer (0.05% UDM, 50 mM Tris-HCl, 150 mM NaCl; pH7.4) and once with 50 µL of 50 mM ammonium acetate buffer. Thereafter, the antibody beads were brought into contact with the first acidic solution (0.05% UDM, 50 mM glycine buffer; pH 2.8) to elute the Ng peptide into the first acidic solution. Accordingly, a first eluate containing the Ng peptide was obtained.

### (Neutralization Step)

The first eluate was mixed with neutral buffer (0.1% UDM, 800 mM GlcNAc, 300 mM Tris-HCl, 300 mM NaCl; pH 7.4) to give a purified solution.

### (Second Bonding Step, Second Washing Step, Second Elution Step)

Purification solution was mixed with antibody beads and incubated at 4°C for 1 h. The antibody beads were then washed twice with 50 µL of the second washing buffer (0.05% UDM, 50 mM Tris-HCl, 150 mM NaCl; pH 7.4), washed once with 50 µL of 50 mM ammonium acetate buffer, and washed once with 30 µL of water. Thereafter, the antibody beads were brought into contact with a second acidic solution (70% (v/v) acetonitrile aqueous solution containing 5 mM hydrochloric acid and 0.1 mM methionine, 50 nM BSA) to elute the Ng peptide into the second acidic solution. Accordingly, a second eluate containing the Ng peptide was obtained.

### [Mass Spectrometry]

AXIMA Performance (Shimadzu/KRATOS, Manchester, UK), which is a MALDI-TOF MS apparatus, was used as a mass spectrometer. Using α-cyano-4-hydroxycinnamic acid (CHCA) as a matrix for Linear TOF, methylene diphosphonic acid (MDPNA) as a matrix additive, and acetonitrile as a solvent, a 2 mg/mL CHCA/0.2% (w/v) MDPNA matrix solution was prepared. Then, 0.5 µL each of the matrix solution was added dropwise to 4 wells of a MALDI plate (µFocus MALDI plate 900 µm(Hudson Surface Technology, Inc., Fort Lee, NJ)) and dried, and then 1 µL each of the second eluent was further added dropwise thereto and dried.

MALDI-TOF MS was then activated to detect the Ng peptide. As a setting condition, mass spectrum data was acquired by Linear TOF in a positive ion mode. For 1 well, 400 spots and 16000 shots were accumulated. The m/z value of Linear TOF was expressed as the peak average mass. The m/z values were calibrated using human angiotensin II and human ACTH fragment 18-39, bovine insulin oxidized beta-chain, bovine insulin, and cytochrome c as external standards.

Tables 3 and 4 show the Ng peptides detected in the mass spectrum. * in the table represents a divalent ion peak. Abbreviations in the table are as follows: SIL: stable isotope-labeled, Ac: acetylation, Cys: cysteinylation, Glu: glutathionylation, and ss: internal disulfide bond. Note that acetylation occurs at the amino acid corresponding to the first of Ng1-78, glutathionylation occurs at the amino acid corresponding to the third or ninth of Ng1-78, and an internal disulfide bond is formed between the amino acids corresponding to the third and fourth of Ng1-78 or between the amino acids corresponding to the fourth and ninth of Ng1-78 (see Non-Patent Document 5).

**[Table 3]**

| Peptide name | Sequence | Theoretical average m/z | No. |
|---|---|---|---|
| Ng43-75* | RKKIKSGE RGRKGPGPGG PGGAGVARGG AGGGP | 1493.2 | 13 |
| Ng43-78* | RKKIKSGE RGRKGPGPGG PGGAGVARGG AGGGPSGD | 1622.8 | 5 |
| Ng53-75 | RKGPGPGG PGGAGVARGG AGGGP | 1845.0 | 1 |
| Ng52-75 | GRKGPGPGG PGGAGVARGG AGGGP | 1902.1 | 6 |
| Ng51-75 | RGRKGPGPGG PGGAGVARGG AGGGP | 2058.3 | 7 |
| Ng53-78 | RKGPGPGG PGGAGVARGG AGGGPSGD | 2104.3 | 2 |
| Ng50-75 | E RGRKGPGPGG PGGAGVARGG AGGGP | 2187.4 | 8 |
| Ng43-65 | RKKIKSGE RGRKGPGPGG PGGAG | 2205.5 | 9 |
| Ng48-75 | SGE RGRKGPGPGG PGGAGVARGG AGGGP | 2331.5 | 3 |
| Ng42-66 | ARKKIKSGE RGRKGPGPGG PGGAGV | 2375.8 | 10 |
| Ng50-78 | E RGRKGPGPGG PGGAGVARGG AGGGPSGD | 2446.6 | 11 |
| Ng45-75 | KIKSGE RGRKGPGPGG PGGAGVARGG AGGGP | 2701.0 | 12 |
| SIL-Ng45-75 | KIKSGE RGRKGPGPGG PGGAGVARGG AGGGP | 2733.1 | 12 |
| Ng44-75 | KKIKSGE RGRKGPGPGG PGGAGVARGG AGGGP | 2829.2 | 4 |
| Ng43-75 | RKKIKSGE RGRKGPGPGG PGGAGVARGG AGGGP | 2985.4 | 13 |
| Ng43-77 | RKKIKSGE RGRKGPGPGG PGGAGVARGG AGGGPSG | 3129.5 | 14 |
| Ng42-77 | ARKKIKSGE RGRKGPGPGG PGGAGVARGG AGGGPSG | 3200.6 | 15 |
| Ng43-78 | RKKIKSGE RGRKGPGPGG PGGAGVARGG AGGGPSGD | 3244.6 | 5 |
| Ng42-78 | ARKKIKSGE RGRKGPGPGG PGGAGVARGG AGGGPSGD | 3315.7 | 16 |
| Ng40-75 | H MARKKIKSGE RGRKGPGPGG PGGAGVARGG AGGGP | 3324.8 | 17 |
| Ng37-70 | FRGH MARKKIKSGE RGRKGPGPGG PGGAGVARGG | 3345.9 | 18 |
| Ng38-75 | RGH MARKKIKSGE RGRKGPGPGG PGGAGVARGG AGGGP | 3538.1 | 19 |

**[Table 4]**

| Peptide name | Sequence | Theoretical average m/z | No. |
|---|---|---|---|
| AcNg1-75ss* | MDCCTENACS KPDDDILDIP LDDPGANAAA AKIQASFRGH MARKKIKSGE RGRKGPGPGG PGGAGVARGG AGGGP | 3700.7 | 28 |
| AcCysNg1-75ss* | MDCCTENACS KPDDDILDIP LDDPGANAAA AKIQASFRGH MARKKIKSGE RGRKGPGPGG PGGAGVARGG AGGGP | 3760.2 | 29 |
| GluNg2-75* | DCCTENACS KPDDDILDIP LDDPGANAAA AKIQASFRGH MARKKIKSGE RGRKGPGPGG PGGAGVARGG AGGGP | 3767.7 | 35 |
| Ng38-78 | RGH MARKKIKSGE RGRKGPGPGG PGGAGVARGG AGGGPSGD | 3797.3 | 20 |
| AcNg1-78ss* | MDCCTENACS KPDDDILDIP LDDPGANAAA AKIQASFRGH MARKKIKSGE RGRKGPGPGG PGGAGVARGG AGGGPSGD | 3830.3 | 30 |
| AcCysNg1-78ss* | MDCCTENACS KPDDDILDIP LDDPGANAAA AKIQASFRGH MARKKIKSGE RGRKGPGPGG PGGAGVARGG AGGGPSGD | 3889.8 | 31 |
| AcGluNg2-78ss* | DCCTENACS KPDDDILDIP LDDPGANAAA AKIQASFRGH MARKKIKSGE RGRKGPGPGG PGGAGVARGG AGGGPSGD | 3917.3 | 32 |
| AcGluNg1-78ss* | MDCCTENACS KPDDDILDIP LDDPGANAAA AKIQASFRGH MARKKIKSGE RGRKGPGPGG PGGAGVARGG AGGGPSGD | 3982.9 | 33 |
| Ng36-78 | SFRGH MARKKIKSGE RGRKGPGPGG PGGAGVARGG AGGGPSGD | 4031.5 | 21 |
| Ng33-75 | IQASFRGH MARKKIKSGE RGRKGPGPGG PGGAGVARGG AGGGP | 4084.7 | 22 |
| Ng33-78 | IQASFRGH MARKKIKSGE RGRKGPGPGG PGGAGVARGG AGGGPSGD | 4343.9 | 23 |
| Ng24-75 | PGANAAA AKIQASFRGH MARKKIKSGE RGRKGPGPGG PGGAGVARGG AGGGP | 4836.5 | 24 |
| SIL-Ng24-75 | PGANAAA AKIQASFRGH MARKKIKSGE RGRKGPGPGG PGGAGVARGG AGGGP | 4868.6 | 24 |
| Recombinant Ng1-78ss* | GSSHHHHHHS SGLVPRGSHM GSMDCCTENACS KPDDDILDIP LDDPGANAAA AKIQASFRGH MARKKIKSGE RGRKGPGPGG PGGAGVARGG AGGGPSGD | 4963.0 | 34 |
| Ng24-78 | PGANAAA AKIQASFRGH MARKKIKSGE RGRKGPGPGG PGGAGVARGG AGGGPSGD | 5095.7 | 25 |
| Ng7-55 | NACS KPDDDILDIP LDDPGANAAA AKIQASFRGH MARKKIKSGE RGRKGPGPGG PGGAGVARGG AGGGP | 5192.9 | 26 |
| Ng23-78 | DPGANAAA AKIQASFRGH MARKKIKSGE RGRKGPGPGG PGGAGVARGG AGGGP | 5210.8 | 27 |
| AcNg1-75ss | MDCCTENACS KPDDDILDIP LDDPGANAAA AKIQASFRGH MARKKIKSGE RGRKGPGPGG PGGAGVARGG AGGGP | 7400.3 | 28 |
| AcCysNg1-75ss | MDCCTENACS KPDDDILDIP LDDPGANAAA AKIQASFRGH MARKKIKSGE RGRKGPGPGG PGGAGVARGG AGGGP | 7519.5 | 29 |
| AcNg1-78ss | MDCCTENACS KPDDDILDIP LDDPGANAAA AKIQASFRGH MARKKIKSGE RGRKGPGPGG PGGAGVARGG AGGGPSGD | 7659.5 | 30 |
| AcCysNg1-78ss | MDCCTENACS KPDDDILDIP LDDPGANAAA AKIQASFRGH MARKKIKSGE RGRKGPGPGG PGGAGVARGG AGGGPSGD | 7778.7 | 31 |
| AcGluNg2-78ss | DCCTENACS KPDDDILDIP LDDPGANAAA AKIQASFRGH MARKKIKSGE RGRKGPGPGG PGGAGVARGG AGGGPSGD | 7793.6 | 32 |
| AcGluNg1-78ss | MDCCTENACS KPDDDILDIP LDDPGANAAA AKIQASFRGH MARKKIKSGE RGRKGPGPGG PGGAGVARGG AGGGPSGD | 7964.9 | 33 |
| Recombinant Ng1-78ss | GSSHHHHHHS SGLVPRGSHM GSMDCCTENACS KPDDDILDIP LDDPGANAAA AKIQASFRGH MARKKIKSGE RGRKGPGPGG PGGAGVARGG AGGGPSGD | 9925.0 | 34 |

### [Measured Value of Ng Peptide]

The peak intensity (NI: Normalized intensity) of each Ng peptide normalized (normalized) with the internal standard peptide (SIL-Ng45-75 or SIL-Ng24-75) was calculated in each of the four spectra for one sample, and the value obtained by averaging the four peak intensities was used as the measured value of the Ng peptide of each sample. Peak intensities (NI) that did not reach the detection limit (S/N<3) were excluded. The number of data used for averaging the peak intensity (NI) was up to four, but when the number of data was less than three, the peak intensity (NI) was determined to be undetectable and defined as 0.

The reproducibility of peak intensity (NI) was evaluated with commercially available plasma spiked with Ng peptide and recombinant Ng. As a result, with respect to the interday reproducibility in 4 days, Ng53-75 had 2.3%CV, Ng48-75 had 7.8%CV, Ng45-75 had 6.9%CV, Ng43-75 had 5.2%CV, Ng24-75 had 3.7%CV, the divalent ion of recombinant Ng had 9.8%CV, and the monovalent ion of recombinant Ng had 15.7%CV. Regarding the intra-assay reproducibility, 4 batches were evaluated, and Ng53-75 had 4.9 to 9.7% CV, Ng48-75 had 3.4 to 6.2% CV, Ng45-75 had 2.1 to 5.5% CV, Ng43-75 had 4.1 to 11.2% CV, Ng24-75 had 1.8 to 7.1% CV, the divalent ion of recombinant Ng had 4.6 to 14.5% CV, and the monovalent ion of recombinant Ng had 3.4 to 10.3% CV. This method had an analytical precision of 20% CV or less and exhibited acceptable reproducibility.

Among the Ng peptides detected in Table 3 and Table 4, 21 Ng peptides detected in 80% or more of the 161 samples, and a total of 28 Ng peptide groups (see Table 5) of 5 [Ngx-75+Ngx-78], Ng protein*, and total Ng protein were analyzed for biomarker search.

Note that, for the Ng peptide in which both monovalent ions and divalent ions are detected, the same Ng peptide is measured in both cases. Therefore, monovalent ions were analyzed for small Ng peptides having a mass of less than 5000, and divalent ions were analyzed for large Ng peptides having a mass of 5000 or more.

### [Correction of Ng Peptide Value in Plasma]

Among the 161 samples, samples obtained by thawing cryopreserved samples, placing the samples at room temperature for about 2 hours, and then re-freezing the samples are also included. In order to investigate the influence of this process on the measured value of the Ng peptide, the Ng peptide to be analyzed was evaluated using a part of the sample. In the measurement of the sample that was thawed, left at room temperature for about 2 hours, and then re-frozen (sample under the RT2h-FT1 condition), the above [purification] [mass spectrometry] was performed except that SIL-Ng50-78 was used as the internal standard peptide, and the internal standard peptide was diluted with the first buffer. The above [purification] [mass spectrometry] was also performed on the samples not subjected to the RT2h-FT1 process (samples under control conditions). For these, data processing was performed using data obtained by simultaneously measuring SIL-Ng50-78 and SIL-Ng45-75 and SIL-Ng24-75 such that the measured value of the RT2h-FT1 condition normalized with SIL-Ng50-78 was NI normalized with SIL-Ng45-75 and SIL-Ng24-75.

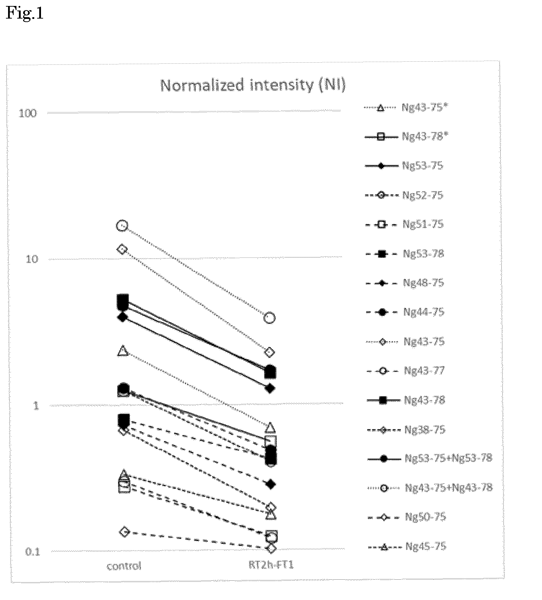

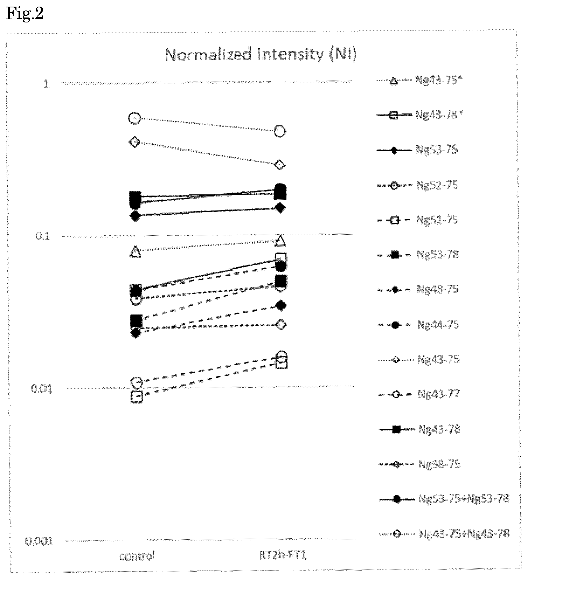

As compared with the samples under the Control condition, the samples under the RT2h-FT1 condition, the NI of the Ng peptide of not more than the mass of Ng38-75 was uniformly decreased (FIG. 1). From this, it can be seen that the decomposition of the Ng peptide proceeds by the time for which the plasma is stored in a thawed state and/or freeze-thawing. Therefore, correction was performed by dividing the peak intensity (NI) of each Ng peptide by the total value (correction coefficient) obtained by adding the peak intensity (NI) of monovalent ions of the 10 Ng peptide (Ng53-75, Ng52-75, Ng51-75, Ng53-78, Ng48-75, Ng44-75, Ng43-75, Ng43-77, Ng43-78, Ng38-75) and the peak intensity (NI) of divalent ions of the 2Ng peptide (Ng43-75, Ng43-78). As a result, the difference between the Ng peptide values (corrected NI) of the sample under the control condition and the sample under the RT2h-FT1 condition was small (FIG. 2). That is, it was shown to be effective in reducing the variation due to the handling method (storage conditions) of the plasma samples.

Since Ng50-75 and Ng45-75 had a smaller decrease width due to decomposition than the other Ng peptides, the difference between the measured values was not reduced by the correction processing, and thus the correction processing was not performed for Ng50-75 and Ng45-75. Therefore, these two kinds of Ng peptides were subjected to the analysis described later with the values not subjected to the correction treatment, and the other Ng peptides were subjected to the analysis described later with the values subjected to the correction treatment.

### [Analysis of Ng Peptide Biomarker]

The medians of the 28 Ng peptide biomarkers to be analyzed in the CN group, the MCI group, the AD_Aβ+ group, and the nonAD_Aβ- group were calculated. This is shown in Table 5.

**[Table 5]**

| Name | Number of detections | Median for each group | | | |
|---|---|---|---|---|---|
| | | CN | MCI | AD | nonAD |
| Ng53-75 | 160 | 0.083 | 0.114 | 0.158 | 0.142 |
| Ng52-75 | 160 | 0.028 | 0.037 | 0.028 | 0.027 |
| Ng51-75 | 135 | 0.009 | 0.009 | 0.008 | 0.007 |
| Ng53-78 | 153 | 0.014 | 0.019 | 0.027 | 0.025 |
| Ng50-75 | 135 | 0.143 | 0.139 | 0.130 | 0.120 |
| Ng48-75 | 160 | 0.034 | 0.034 | 0.019 | 0.023 |
| Ng45-75 | 159 | 0.282 | 0.320 | 0.236 | 0.259 |
| Ng44-75 | 160 | 0.104 | 0.097 | 0.045 | 0.058 |
| Ng43-75 | 160 | 0.473 | 0.436 | 0.359 | 0.362 |
| Ng43-77 | 130 | 0.009 | 0.009 | 0.011 | 0.011 |
| Ng43-78 | 159 | 0.074 | 0.113 | 0.201 | 0.189 |
| Ng38-75 | 129 | 0.014 | 0.016 | 0.016 | 0.017 |
| AcNg1-75ss* | 161 | 1.219 | 0.858 | 0.771 | 0.860 |
| AcCysNg1-75ss* | 161 | 3.344 | 2.238 | 2.084 | 2.447 |
| AcNg1-78ss* | 161 | 1.678 | 1.332 | 1.772 | 1.739 |
| AcCysNg1-78ss* | 161 | 3.880 | 3.233 | 4.131 | 4.179 |
| AcGluNg2-78ss* | 155 | 0.265 | 0.224 | 0.284 | 0.284 |
| AcGluNg1-78ss* | 160 | 0.583 | 0.368 | 0.567 | 0.535 |
| Ng24-75 | 143 | 0.244 | 0.195 | 0.146 | 0.160 |
| Ng24-78 | 159 | 0.267 | 0.238 | 0.299 | 0.244 |
| Ng7-55 | 159 | 0.137 | 0.126 | 0.152 | 0.140 |
| Ng53-75+Ng53-78 | 160 | 0.100 | 0.123 | 0.209 | 0.181 |
| Ng43-75+Ng43-78 | 160 | 0.570 | 0.554 | 0.527 | 0.569 |
| AcNg1-75ss*+AcNg1-78ss* | 161 | 2.984 | 2.694 | 2.643 | 2.599 |
| AcCysNg1-75ss*+AcCysNg1-78ss* | 161 | 7.403 | 7.472 | 6.398 | 6.383 |
| Ng24-75+Ng24-78 | 160 | 0.518 | 0.521 | 0.422 | 0.416 |
| Ng proteins* | 161 | 11.169 | 10.790 | 10.132 | 9.608 |
| total Ng proteins | 161 | 20.293 | 19.689 | 17.587 | 17.912 |

The Ng peptide biomarkers with higher median values in the AD_Aβ+ group than in the CN group were Ng53-75, Ng53-78, Ng43-77, Ng43-78, Ng38-75, AcNg1-78ss*, AcCysNg1-78ss*, AcGluNg2-78ss*, Ng24-78, Ng7-55, Ng53-75+Ng53-78. The Ng peptide biomarkers with lower median values in the AD_AB+ group than in the CN group were Ng52-75, Ng51-75, Ng50-75, Ng48-75, Ng45-75, Ng44-75, Ng43-75, AcNg1-75ss*, AcCysNg1-75ss*, AcGluNg1-78ss*, Ng24-75, Ng43-75+Ng43-78, AcNg1-75ss*+AcNg1-78ss*, AcCysNg1-75ss*+AcCysNg1-78ss*, Ng24-75+Ng24-78, Ng proteins*, total Ng proteins. The Ng peptide biomarkers with higher median values in the nonAD_AB- group than in the CN group were Ng53-75, Ng53-78, Ng43-77, Ng43-78, Ng38-75, AcNg1-78ss*, AcCysNg1-78ss*, AcGluNg2-78ss*, Ng7-55, and Ng53-75+Ng53-78. The Ng peptide biomarkers with lower median values in the nonAD_Aβ- group than in the CN group were Ng52-75, Ng51-75, Ng50-75, Ng48-75, Ng45-75, Ng44-75, Ng43-75, AcNg1-75ss*, AcCysNg1-75ss*, AcGluNg1-78ss*, Ng24-75, Ng24-78, AcNg1-75ss*+AcNg1-78ss*, AcCysNg1-75ss*+AcCysNg1-78ss*, Ng24-75+Ng24-78, Ng proteins*, total Ng proteins.

AD_Aβ+ or nonAD_Aβ- is plasma derived from a subject who has been diagnosed with dementia and whose cognitive function has deteriorated, and thus has already undergone neurodegeneration of the brain. Therefore, it can be seen that when AD_Aβ+ or nonAD_Aβ- is increased or decreased in comparison with CN group, it can be used as a biomarker that varies reflecting neurodegeneration of the brain.

Of the 28 Ng peptide biomarkers to be analyzed, 9 Ng peptide biomarkers met the criteria as a result of evaluation using the following screening criteria (Table 6). In the table, the underline indicates the Ng peptide biomarker satisfying the marker screening criteria.

### [Screening Criteria]

The median of AD_Aβ+ or nonAD_Aβ- is more than 1.5 times or less than 0.666 times CN.

**[Table 6]**

| Name | Number of detections | Difference in median for each group | | |
|---|---|---|---|---|
| | | CN vs MCI | CN vs AD_A*β* + | CN vs nonAD_A*β* - |
| Ng53-75 | 160 | 1.38 | 1.91 | 1.72 |
| Ng52-75 | 160 | 1.30 | 0.98 | 0.98 |
| Ng51-75 | 135 | 1.08 | 0.94 | 0.86 |
| Ng53-78 | 153 | 1.39 | 1.98 | 1.85 |
| Ng50-75 | 135 | 0.97 | 0.91 | 0.84 |
| Ng48-75 | 160 | 1.02 | 0.55 | 0.68 |
| Ng45-75 | 159 | 1.14 | 0.84 | 0.92 |
| Ng44-75 | 160 | 0.93 | 0.43 | 0.56 |
| Ng43-75 | 160 | 0.92 | 0.76 | 0.77 |
| Ng43-77 | 130 | 1.07 | 1.32 | 1.31 |
| Ng43-78 | 159 | 1.53 | 2.72 | 2.56 |
| Ng38-75 | 129 | 1.10 | 1.13 | 1.17 |
| AcNg1-75ss* | 161 | 0.70 | 0.63 | 0.71 |
| AcCysNg1-75ss* | 161 | 0.67 | 0.62 | 0.73 |
| AcNg1-78ss* | 161 | 0.79 | 1.06 | 1.04 |
| AcCysNg1-78ss* | 161 | 0.83 | 1.06 | 1.08 |
| AcGluNg2-78ss* | 155 | 0.84 | 1.07 | 1.07 |
| AcGluNg1-78ss* | 160 | 0.63 | 0.97 | 0.92 |
| Ng24-75 | 143 | 0.80 | 0.60 | 0.65 |
| Ng24-78 | 159 | 0.89 | 1.12 | 0.91 |
| Ng7-55 | 159 | 0.92 | 1.11 | 1.02 |
| Ng53-75+Ng53-78 | 160 | 1.23 | 2.10 | 1.81 |
| Ng43-75+Ng43-78 | 161 | 0.97 | 0.92 | 1.00 |
| AcNg1-75ss*+AcNg1-78ss* | 161 | 0.90 | 0.89 | 0.87 |
| AcCysNg1-75ss*+AcCysNg1-78ss* | 160 | 1.01 | 0.86 | 0.86 |
| Ng24-75+Ng24-78 | 161 | 1.01 | 0.82 | 0.80 |
| Ng proteins* | 161 | 0.97 | 0.91 | 0.86 |
| total Ng proteins | 160 | 0.97 | 0.87 | 0.88 |

One-way ANOVA of Kruskal-Wallis test and Holm corrected Wilcoxon's rank-sum test were performed to confirm whether the difference in the median of the 9 Ng peptide biomarkers between groups was statistically significant. The results showed that the differences between the AD_AB+ group and the CN group and between the nonAD_Aβ-group and the CN group were significant for all 9 Ng peptide biomarkers (Table 7).

**[Table 7]**

| | Test of difference in median for each group of 11 Ng peptides meeting marker screening criteria (p-value, significance level p < 0.05) | | | |
|---|---|---|---|---|
| | Kruskal-Wallis Test (one-way ANOVA) | Wilcoxon rank-sum test (Holm correction) | | |
| | | CN vs MCI | CN vs AD_A*β* + | CN vs nonAD_A*β* - |
| Ng53-75 | <.0001 | 0.0509 | <0.0001 | <0.0001 |
| Ng53-78 | <.0001 | 0.3627 | <0.0001 | <0.0001 |
| Ng48-75 | <.0001 | 0.7003 | <0.0001 | <0.0001 |
| Ng44-75 | <.0001 | 0.2540 | <0.0001 | <0.0001 |
| Ng43-78 | <.0001 | 0.1361 | <0.0001 | <0.0001 |
| AcNgl-75ss* | 0.0093 | 0.3377 | 0.0113 | 0.0114 |
| AcCysNg1-75ss* | 0.0143 | 0.5062 | 0.0160 | 0.0170 |
| Ng24-75 | 0.0022 | 0.4710 | 0.0025 | 0.0065 |
| Ng53-75+Ng53-78 | <.0001 | 0.0699 | <0.0001 | <0.0001 |

Further, ROC analysis was performed to evaluate the ability to detect AD_Aβ+ or nonAD_Aβ-. As a result, it was found that there were 6 types of Ng peptide biomarkers showing good AUC in total including Ng53-75, Ng53-78, Ng48-75, Ng44-75, Ng43-78, and Ng53-75+Ng53-78 (combination of Ng53-75 and Ng53-78) (Table 8). On the other hand, in three types of AcNg1-75ss*, AcCysNg1-75ss*, and Ng24-75, the AUC was less than 0.7, and thus the accuracy of the biomarker was low.

**[Table 8]**

| | AUC in ROC analysis | | |
|---|---|---|---|
| | CN vs MCI | CN vs AD_A*β+* + | CN vs nonAD_A*β* - |
| Ng53-75 | 0.674 | 0.880 | 0.799 |
| Ng53-78 | 0.580 | 0.811 | 0.750 |
| Ng48-75 | 0.535 | 0.755 | 0.742 |
| Ng44-75 | 0.599 | 0.840 | 0.785 |
| Ng43-78 | 0.633 | 0.848 | 0.844 |
| AcNg1-75ss* | 0.586 | 0.672 | 0.661 |
| AcCysNg1-75ss* | 0.559 | 0.665 | 0.654 |
| Ng24-75 | 0.565 | 0.698 | 0.672 |
| Ng53-75+Ng53-78 | 0.662 | 0.888 | 0.818 |

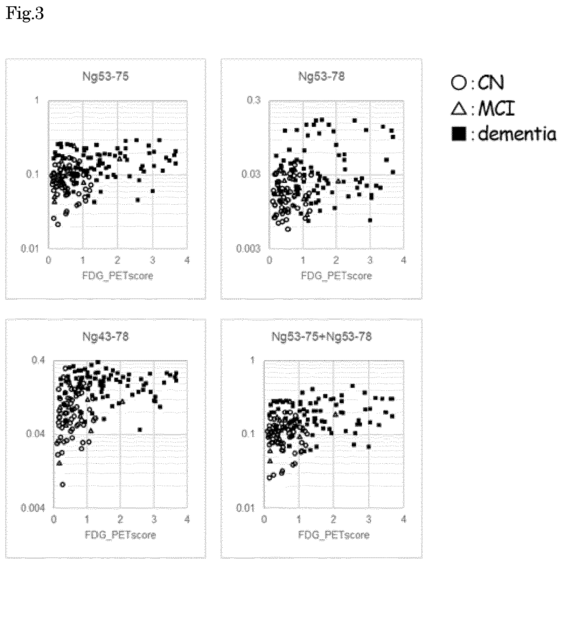

The association of these six Ng peptide biomarkers with FDG-PET, VSRAD and MMSE is shown in Figs 3 to 8. MMSE is an examination score for evaluating cognitive function, FDG-PET is a score indicating functional degradation of nerve cells and synapses in the brain by evaluating brain carbohydrate metabolism, and VSRAD is a score indicating brain atrophy caused by falling of nerve cells in the medial temporal lobe by MRI examination, and these are used as indices of neurodegeneration in the brain. The higher the FDG-PET and VSRAD, the more advanced the neurodegeneration is, and the lower the MMSE, the more advanced the neurodegeneration is.

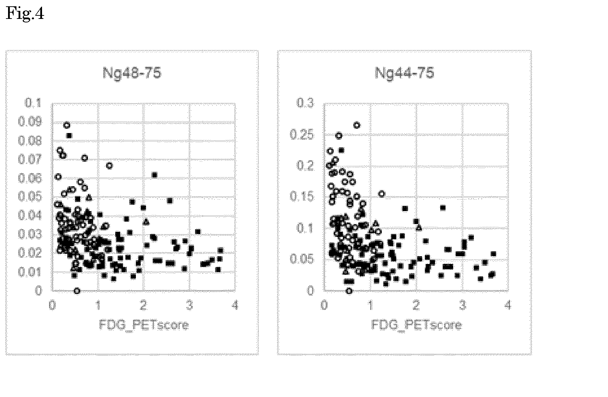

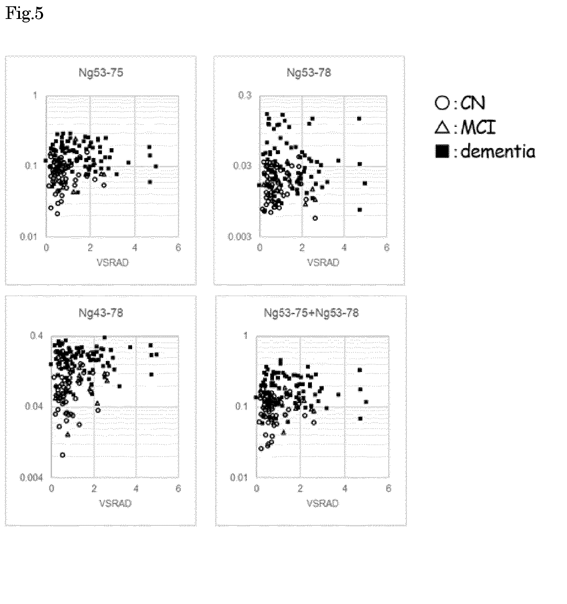

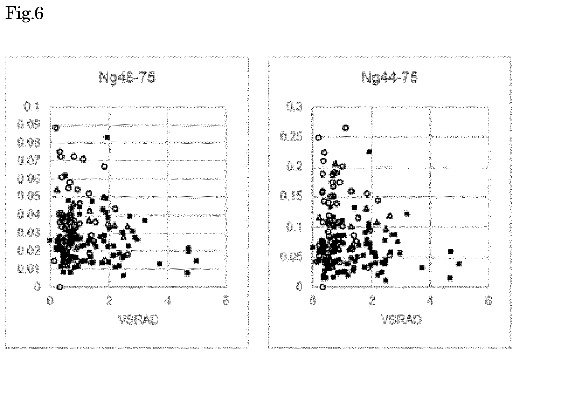

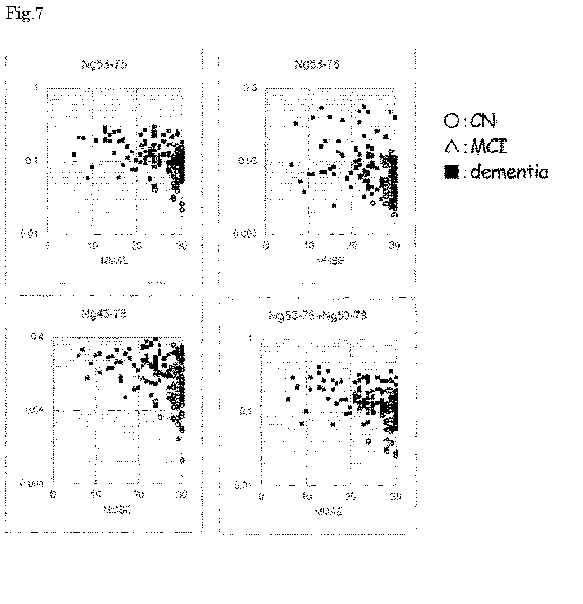

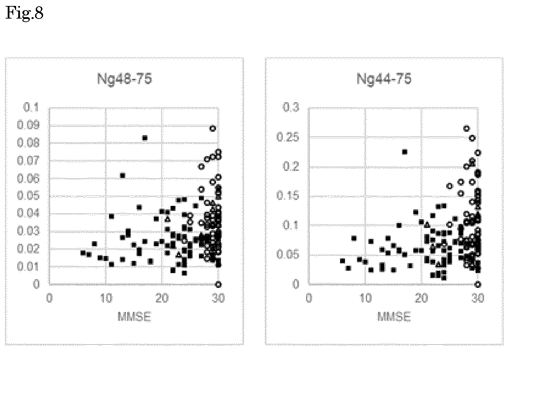

In the relationship between each Ng peptide biomarker and FDG-PET, VSRAD, or MMSE, in a case where FDG-PET or VSRAD shows a high value, Ng53-78, Ng53-78, Ng43-78, and Ng53-75+Ng53-78 tend to be high (FIGS. 3 and 5), while Ng48-75 and Ng44-75 tend to be low (FIGS. 4 and 6). Similarly, even in the case where the MMSE is low, Ng53-78, Ng53-78, Ng43-78, and Ng53-75+Ng53-78 tend to be high (FIG. 7), while Ng48-75 and Ng44-75 tend to be low (FIG. 8). These results indicate that these Ng peptides capture neurodegenerative changes in the brain that can be detected with FDG-PET, VSRAD, and MMSE. Also in Dementia cases that could not be captured by FDG-PET, VSRAD, or MMSE, Ng53-78, Ng53-78, Ng43-78, and Ng53-75+Ng53-78 showed a high tendency, and Ng48-75 and Ng44-75 showed a low tendency. This indicates that neurodegeneration of the brain of Dementia cases that are overlooked by FDG-PET, VSRAD, and MMSE is also captured.

Although the difference between the CN group and the AD_Aβ+ or nonAD_Aβ-group may be a difference caused by the difference between the AB positive and AB negative as AD pathology, there was no statistically significant difference in the difference in the median value between the AB positive group and the AB negative group in the CN group and the difference in the median value between the AB positive group and the AB negative group in the case group developing dementia, that is, between the AD_Aβ+ group and the nonAD_AB- group (Table 9).

From the above, it can be seen that the six Ng peptide biomarkers do not vary reflecting only AD pathology, but reflect dementia due to neurodegeneration (including synaptic disorder) of the brain.

**[Table 9]**

| Test of difference in median between Aβ positive and Aβ negative (p-value, significance level p < 0.05) | | |
|---|---|---|
| | Wilcoxon rank-sum test (Holm correction) | |
| | CN_A*β* - vs CN_A*β* + | nonAD_A*β* - vs AD_A*β* + |
| Ng53-75 | 0.3873 | 0.4805 |
| Ng53-78 | 0.2991 | 0.3876 |
| Ng48-75 | 0.0587 | 0.6099 |
| Ng44-75 | 0.1738 | 0.1144 |
| Ng43-78 | 1.0000 | 0.8852 |
| Ng53-75+Ng53-78 | 0.3968 | 0.2117 |

### <Experimental Example 2>

### [Change of Ng Peptide Group Serving as Correction Coefficient]

(2-1) The Ng peptides (10 monovalent ions and 2 divalent ions) subject to the correction coefficient were changed to the monovalent ions of the 12 Ng peptides (Ng53-75, Ng52-75, Ng51-75, Ng53-78, Ng48-75, Ng44-75, Ng43-75, Ng43-77, Ng43-78, Ng38-75, Ng50-75, and Ng45-75) and the divalent ions of the 2 Ng peptides (Ng43-75 and Ng43-78), and correction processing was performed with the sum of these peak intensities. Except for this correction processing, the difference in the median values of the Ng peptide biomarkers was calculated in the same manner as in Experimental Example 1. The results are shown in Table 10. Also in this case, it was found that the above marker screening criteria were satisfied. Similarly, it was confirmed by one-way analysis of variance of Kruskal-Wallis test and the like that the difference was statistically significant, and it was confirmed by ROC analysis that a good AUC was shown.

**[Table 10]**

| Ng peptide | Number of detections | Difference in median for each group | | |
|---|---|---|---|---|
| | | CN vs MCI | CN vs AD_A*β*+ | CN vs nonAD_A*β* - |
| Ng53-75 | 160 | 1.42 | 2.00 | 1.79 |
| Ng53-78 | 153 | 1.41 | 2.06 | 1.85 |
| Ng48-75 | 160 | 1.01 | 0.57 | 0.68 |
| Ng44-75 | 160 | 0.93 | 0.44 | 0.56 |
| Ng43-78 | 159 | 1.60 | 2.94 | 2.79 |
| Ng53-75+Ng53-78 | 160 | 1.26 | 2.16 | 1.87 |

(2-2) The Ng peptides (10 monovalent ions and 2 divalent ions) subject to the correction coefficient were changed to the monovalent ions of the 10 Ng peptides (Ng53-75, Ng52-75, Ng51-75, Ng53-78, Ng48-75, Ng44-75, Ng43-75, Ng43-77, Ng43-78, and Ng38-75), and correction processing was performed with the sum of these peak intensities. Except for this correction processing, the difference in the median values of the Ng peptide biomarkers was calculated in the same manner as in Experimental Example 1. The results are shown in Table 11. Also in this case, it was found that the above marker screening criteria were satisfied. Similarly, it was confirmed by one-way analysis of variance of Kruskal-Wallis test and the like that the difference was statistically significant, and it was confirmed by ROC analysis that a good AUC was shown.

**[Table 11]**

| Ng peptide | Number of detections | Difference in median for each group | | |
|---|---|---|---|---|
| | | CN vs MCI | CN vs AD_A*β* + | CN vs nonAD_A*β* - |
| Ng53-75 | 160 | 1.43 | 1.96 | 1.75 |
| Ng53-78 | 153 | 1.37 | 1.96 | 1.81 |
| Ng48-75 | 160 | 1.00 | 0.53 | 0.65 |
| Ng44-75 | 160 | 0.97 | 0.43 | 0.55 |
| Ng43-78 | 159 | 1.60 | 2.81 | 2.55 |
| Ng53-75+Ng53-78 | 160 | 1.25 | 2.08 | 1.75 |

(2-3) The Ng peptides (10 monovalent ions and 2 divalent ions) subject to the correction coefficient were changed to the monovalent ions of the 2 Ng peptides (Ng52-75 and Ng38-75), and correction processing was performed with the sum of these peak intensities. Except for this correction processing, the difference in the median values of the Ng peptide biomarkers was calculated in the same manner as in Experimental Example 1. The results are shown in Table 12. In this case, it was shown that the above marker screening criteria were met (Table 12). Similarly, it was confirmed by one-way analysis of variance of Kruskal-Wallis test and the like that the difference was statistically significant, and it was confirmed by ROC analysis that a good AUC was shown.

**[Table 12]**

| Ng peptide | Number of detections | Difference in median for each group | | |
|---|---|---|---|---|
| | | CN vs MCI | CN vs AD_A*β* + | CN vs nonAD_A*β* - |
| Ng53-75 | 160 | 1.15 | 2.16 | 1.86 |
| Ng53-78 | 153 | 0.86 | 1.90 | 1.97 |
| Ng48-75 | 160 | 0.81 | 0.58 | 0.69 |
| Ng44-75 | 160 | 0.85 | 0.40 | 0.60 |
| Ng43-78 | 159 | 1.39 | 2.78 | 3.20 |
| Ng53-75+Ng53-78 | 160 | 1.02 | 2.32 | 1.73 |

From the above, it was found that the Ng peptide to be subjected to the correction factor is not limited to a monovalent ion of 10Ng peptide (Ng53-75, Ng52-75, Ng51-75, Ng53-78, Ng48-75, Ng44-75, Ng43-75, Ng43-77, Ng43-78, Ng38-75) and a divalent ion of 2Ng peptide (Ng43-75, Ng43-78), and may be at least two kinds selected from Ng peptides having a mass smaller than that of Ng38-75.

### <Experimental Example 3>

The difference in the median of the Ng peptide biomarkers was calculated in the same manner as in Experimental Example 1 except that no correction was made. The results are shown in Table 13. Also in this case, it was found that the above marker screening criteria were satisfied. Similarly, it was confirmed by one-way analysis of variance of Kruskal-Wallis test and the like that the difference was statistically significant, and it was confirmed by ROC analysis that a good AUC was shown.

**[Table 13]**

| Ng peptide | Number of detections | Difference in median for each group | | |
|---|---|---|---|---|
| | | CN vs MCI | CN vs AD_A*β* + | CN vs nonAD_A*β* - |
| Ng53-75 | 160 | 2.25 | 4.88 | 3.84 |
| Ng53-78 | 153 | 1.67 | 5.49 | 3.71 |
| Ng43-78 | 159 | 2.9 | 7.63 | 5.59 |
| Ng53-75+Ng53-78 | 160 | 2.16 | 4.88 | 4.27 |

### <Experimental Example 4>

### [Multivariate Analysis of Ng Peptide Marker]

The prediction probability of dementia by logistic regression analysis was calculated using 6 Ng peptide, and ROC analysis was performed. As a result, higher AUC was obtained in CN vs AD_Aβ+, CN vs nonAD_Aβ-, and CN vs MCI than in each of the 6 Ng peptide biomarkers (Table 15). In addition, Ng peptides (Ng53-75 + Ng53-78 and Ng44-75) were selected by a stepwise method, and the prediction probability of dementia was calculated using the Ng peptides. Also in this case, higher AUC than any of the 6 Ng peptide biomarkers was obtained (Table 15).

Normalization was performed using the respective average and standard deviation of Ng53-75+Ng53-78 and Ng44-75 of 161 specimens to calculate a z-score, and a synthetic z-score was calculated by subtracting Ng44-75 from Ng53-75 + Ng53-78 (Table 14). As a result, higher AUC than any of the 6 Ng peptide biomarkers was obtained in CN vs AD_Aβ+, CN vs nonAD_Aβ-, and CN vs MCI (Table 15). Similarly, a synthetic z-score obtained by adding the z-score of Ng43-78 and the z-score of Ng53-75 + Ng53-78 was calculated (Table 14). As a result, higher AUC was obtained in CN vs AD_AB+ and CN vs nonAD_Aβ- than in any of the 8 Ng peptide biomarkers (Table 15).

The above results indicate that the ability to detect neurodegeneration of the brain is improved by multivariate analysis.

**[Table 14]**

| Method | | Median between each group of variable calculated by multivariate analysis | | | |
|---|---|---|---|---|---|
| | | CN | MCI | AD_A*β* + | nonAD_A*β* - |
| Logistic regression | 6Ng peptide | 0.184 | 0.498 | 0.924 | 0.875 |
| | 2 Ng peptide | 0.226 | 0.556 | 0.931 | 0.869 |
| Synthetic z-score | [Ng44-75] and [Ng53-75 + Ng53-78] | -1.168 | -0.454 | 1.117 | 0.809 |
| | [Ng43-78] and [Ng 53-75+ Ng 53-78] | -1.570 | -0.865 | 0.936 | 0.839 |

**[Table 15]**

| Method | | AUC in ROC analysis using variables calculated by multivariate analysis | | |
|---|---|---|---|---|
| | | CN vs MCI | CN vs AD_A*β* + | CN vs nonAD_Aβ - |
| Logistic regression | 6Ng peptide | 0.700 | 0.936 | 0.894 |
| | 2 Ng peptide | 0.699 | 0.940 | 0.891 |
| Synthetic z-score | [Ng44-75] and [Ng53-75 + Ng53-78] | 0.683 | 0.938 | 0.889 |
| | [Ng43-78] and [Ng 53-75+ Ng 53-78] | 0.675 | 0.914 | 0.872 |

### <Comparison with Marker of Non-Patent Documents 5 to 7>

Non-Patent Documents 5 to 7 disclose Ng molecules shown in Table 16 as markers. In these documents, comparison between two groups, control (healthy subjects) and AD, has been performed and p-values calculated. This is shown in Table 16. Typically, the larger the difference between the two groups, the lower the p-value. The lowest p-value among the Ng molecules shown in Table 16 was 0.002 with Ng48-76. On the other hand, the difference between the groups of NCvsAD_Aβ+ of the 6 Ng markers, which corresponds to the difference between the 2 groups of control (healthy subjects) and AD, shows p < 0.0001 (Table 7), and it can be said that the difference between the 2 groups is larger than that of the Ng molecules shown in Table 16.

As a test method, Mann-Whitney U test was used in Non-Patent Document 5, Mann-Whitney U test was used in Non-Patent Document 6, and Wilcoxon's rank-sum test was used for the 6 Ng markers. Since Mann-Whitney U test and Wilcoxon's rank-sum test are equivalent, these test methods are used for the markers of the non-patent literature and the six markers. In Table 16, "total FL" is the sum of three peaks of Ng1-78 slightly different in post-translational modification illustrated in FIG. 7c of Non-Patent Document 5.

**[Table 16]**

| Document | Sample | Molecule | Control (healthy subject) vs ADP value |
|---|---|---|---|
| Non-Patent Document 5 | Brain tissue | Ng53-75/total FL ratio | 0.035 |
| | | Ng53-78/total FL ratio | 0.023 |
| | | Ng51-78/total FL ratio | 0.046 |
| | | Ng48-76/total FL ratio | 0.019 |
| | | Ng48-78/total FL ratio | 0.019 |
| | | Ng44-76/total FL ratio | 0.029 |
| | | Ng42-78/total FL ratio | 0.016 |
| | | Ng41-78/total FL ratio | 0.027 |
| Non-Patent Documents 6 and 7 | CSF | Ng48-76 | Study1, 0.002 Study2, between 0.05 and 0.01 |

Among the Ng molecules shown in Table 16, all except Ng53-75 and Ng53-78 should have been detectable (see Tables 3 and 4) in the detection of the Ng molecule in the blood sample shown in Experimental Example 1 if the Ng molecule was present in the sample at a concentration equal to or higher than the detection limit in this measurement method. Therefore, it can be said that molecular markers other than Ng53-75 and Ng53-78 disclosed in Non-Patent Documents 5 to 7 are not effective as markers in blood samples.

## Claims

1. A biomarker for determining a brain neurodegenerative condition, the biomarker comprising at least one selected from the group consisting of Ng53-75, Ng53-78, Ng48-75, Ng44-75, and Ng43-78 in a blood sample.

2. The biomarker according to claim 1, wherein the biomarker is selected from the group consisting of:
Ng53-75,
Ng53-78,
Ng48-75,
Ng44-75,
Ng43-78, and
a combination of Ng53-75 and Ng53-78.

3. An estimation method for a brain neurodegenerative condition, the method comprising:
a measurement step of measuring a blood sample collected from a subject to obtain measured values of a plurality of neurogranin-related peptides; and
a use step of using at least one measured value selected from the group consisting of Ng53-75, Ng53-78, Ng48-75, Ng44-75, and Ng43-78 among the measured values of the plurality of neurogranin-related peptides to determine a brain neurodegenerative condition.

4. The estimation method according to claim 3, wherein the use step includes using at least one measured value selected from the group consisting of:
Ng53-75,
Ng53-78,
Ng48-75,
Ng44-75,
Ng43-78, and
a sum of Ng53-75 and Ng53-78.

5. The estimation method according to claim 3, wherein
the use step includes using a measured value of Ng53-75, Ng53-78, Ng43-78, or a sum of Ng53-75 and Ng53-78, and
when the measured value is higher than a predetermined value, it is determined that neurodegeneration has occurred in the brain of the subject.

6. The estimation method according to claim 3, wherein
the use step includes using Ng48-75 or Ng44-75, and
when the measured value is lower than a predetermined value, it is determined that neurodegeneration has occurred in the brain of the subject.

7. The estimation method according to claim 3, wherein the use step is of comparing a preset reference value for a normal cognitive function with a measured value of the subject obtained in the measurement step.

8. The estimation method according to claim 3, wherein
the measurement step is performed a plurality of times over time, and
the use step is of comparing a plurality of measured values obtained by performing the measurement step a plurality of times.

9. The estimation method according to claim 3, wherein the measured value used in the use step is corrected using a value obtained by adding at least two of the measured values of the plurality of neurogranin-related peptides measured in the measurement step.

10. The estimation method according to claim 3, wherein,
in the use step, at least two measured values are selected from the group consisting of Ng53-75, Ng53-78, Ng48-75, Ng44-75, and Ng43-78 among the measured values of the plurality of neurogranin-related peptides to calculate a variable using multivariate analysis, and
the variable is used to determine a neurodegenerative condition.

11. The estimation method according to claim 3, wherein in the measurement step, mass spectrometry is performed on the blood sample to obtain measured values of peak intensities for the plurality of neurogranin-related peptides.

12. The estimation method according to claim 11, wherein in the measurement step, affinity purification is performed on the blood sample before mass spectrometry.
